(19) Europäisches Patentamt

European Patent Office

Office européen des brevets

(11) **EP 0 759 452 A2**

## (12) EUROPÄISCHE PATENTANMELDUNG

(43) Veröffentlichungstag:
26.02.1997 Patentblatt 1997/09

(51) Int. Cl.$^6$: **C08G 65/30**, C08L 71/02,
C08G 18/48

(21) Anmeldenummer: 96112666.1

(22) Anmeldetag: 06.08.1996

(84) Benannte Vertragsstaaten:
**BE DE ES FR GB IT NL PT**

(30) Priorität: 18.08.1995 DE 19530388

(71) Anmelder: **BAYER AG**
**51368 Leverkusen (DE)**

(72) Erfinder:
- **Gupta, Pramod, Dr.**
  **50181 Bedburg (DE)**
- **Jacobs, Gundolf, Dr.**
  **51503 Rösrath (DE)**
- **Leuridan, Joël, Dr.**
  **2018 Antwerpen (BE)**

(54) **Gereinigte Polyetherpolyole, ein Verfahren zu deren Herstellung sowie deren Verwendung**

(57)  Geruchsarme, höhermolekular Polyetherpolyole werden erhalten, indem man die zu reinigenden Polyetherpolyole unter Zugabe von 5 bis 30 Gew.-% Wasser bei Temperaturen von 110 bis 150°C und Drükken von 10 bis 70 hPa destilliert, wobei man das Wasser in einer Dosierzeit von 1 bis 5 Stunden in feinverteilter Form mit einem Tropfendurchmesser von 5 - 100 µ durch die zu reinigenden Polyetherpolyole leitet.

EP 0 759 452 A2

EP 0 759 452 A2

**Beschreibung**

Die vorliegende Erfindung betrifft geruchsarme, höhermolekulare Polyetherpolyole, ein Verfahren zu deren Herstellung sowie deren Verwendung für die Herstellung von auf Polyetherpolyolen aufbauenden Polymeren, Kosmetika und pharmazeutischen Produkten.

Bei der technischen Herstellung von Polyetherpolyolen durch Alkoxylierung von geeigneten Starterverbindungen mit Zerewitinoff-aktiven Wasserstoffatomen erhält man nach der Neutralisation des Reaktionsgemisches, der Entwässerung und anschließenden Filtration von anorganischen Salzen, Polyetherpolyole, die je nach Verfahrensweise 0,1 bis 2 Gew.-% restliches Wasser, bis zu 2 Gew.-% Lösungsmittel (im allgemeinen ein organisches Lösungsmittel) und Geruchsstoffe, wie Aldehyde, Dioxolane, Dioxane, Allylalkohol sowie Mono-, Di- und Tri-Propylenglykolallylether enthalten. Obwohl diese Geruchsstoffe im allgemeinen nur in geringen Mengen vorhanden sind, verleihen sie jedoch den Polyetherpolyolen einen charakteristischen, intensiven aromatischen Geruch.

In der Technik sind daher verschiedene Reinigungsverfahren entwickelt und beschrieben worden, um die herstellungsbedingten, unerwünschten Nebenprodukte aus den Polyetherpolyolen zu entfernen. So wird beispielsweise in DE-A 2 755 089 ein verbessertes Verfahren zur Entfernung von störenden Nebenprodukten, insbesondere von kleinen Mengen von Wasser und Lösungsmittel sowie von niedermolekularen Glykolen und geruchsintensiven Stoffen mittels eines Schlangenrohrverdampfers beschrieben. Nachteilig bei diesem Verfahren ist jedoch, daß die vorhandenen organischen Lösungsmittel nur unvollständig und die erwähnten geruchsintensiven Stoffe nur geringfügig entfernt werden. Ein weiterer Nachteil des in der genannten deutschen Offenlegungsschrift beschriebenen Verfahrens ist der damit verbundene hohe apparative Aufwand.

Prinzipell können für die Reinigung der Polyetherpolye auch Fallfilmverdampfer eingesetzt werden. Sie weisen jedoch die gleichen Nachteile auf wie das in der genannten deutschen Offenlegungsschrift beschriebene Reinigungsverfahren mittels eines Schlangenrohrverdampfers.

In der japanischen Patentanmeldung 56/104 936 wird ein Verfahren zur Reinigung von Polyetherpolyolen beschrieben, bei dem man bei einen pH-Wert > 6,5 entweder die rohen Polyetherpolyole unter vermindertem Druck bei erhöhter Temperatur destilliert oder in dem man Wasser, Wasserdampf oder Stickstoff ebenfalls bei vermindertem Druck und bei erhöhter Temperatur durch die rohen Polyetherpolyole leitet. Wie das Vergleichsbeispiel 2 der genannten japanischen Patentanmeldung jedoch zeigt, werden beim Durchleiten von Wasser durch die rohe Polyetherpolyol-Mischung die störenden Geruchsstoffe nur unzureichend entfernt. Dies zeigen die entsprechenden Analysenwerte und Geruchsproben. Außerdem waren die nach Vergleichsbeispiel 2 erhaltenen Polyetherpolyole weniger haltbar, d.h. der Aldehydgehalt in den Polyetherpolyolen nahm zu, ebenso wie der unangenehme Geruch.

Aufgabe der vorliegenden Erfindung war es nun, Polyetherpolyole zur Verfügung zu stellen, deren geruchsbildenden Beiprodukte weitestgehend entfernt wurden und die daher praktisch geruchsfrei sind. Solche praktisch geruchsfreien Polyetherpolyole genügen somit den heutigen Anforderungen, insbesondere bei deren Verwendung zur Herstellung von Polyurethanen und PU-Weichschäumen, die wiederum bei der Möbel- oder Matrazenherstellung eingesetzt werden. Außerdem werden in der Kosmetikindustrie und in der pharmazeutischen Industrie hohe Anforderungen an die Reinheit der dort verwendeten Polyetherpolyole gestellt, die die erfindungsgemäßen Polyetherpolyole erfüllen können.

Gegenstand der vorliegenden Erfindung sind daher geruchsarme, monofunktionelle und polyfunktionelle Polyetherpolyole, deren Molekulargewicht bei 750 bis 18 000, bevorzugt bei 1 000 bis 15 000, ganz besonders bevorzugt bei 2 000 bis 12 000, liegt (berechnet nach OH-Zahl und Funktionalität:

$$\text{Mol Gew.} = \frac{\text{F OH.56.100}}{\text{OH-Zahl}} )$$

und deren Viskosität bei 25°C, bestimmt mit Höpplerviskosimeter nach DIN 53015, 40 bis 25 000, vorzugsweise 50 bis 10 000 mPa · s beträgt, die dadurch gekennzeichnet sind, daß deren Gehalt an

a) 2-Methyl-2-Pentenal < 1,5 ppm, bevorzugt < 0,2 ppm,

b) Allylalkohol < 1,0 ppm, bevorzugt < 0,2 ppm,

c) Allyloxypropanol < 15 ppm, bevorzugt < 5,0 ppm,

d) Dipropylenglykolallylether < 50 ppm, bevorzugt < 0,5 ppm,

e) Propionaldehyd < 1,0 ppm, bevorzugt < 0,2 ppm

2

beträgt.

Die erfindungsgemäß beanspruchten Polyetherpolyole werden bekanntlich durch Polymerisation von Epoxiden, wie Ethylenoxid, Propylenoxid, Butylenoxid, Tetrahydrofuran, Styroloxid oder Epichlorhydrin mit sich selbst, beispielsweise in Gegenwart von Säuren oder durch Anlagerung dieser Epoxide, gegebenenfalls im Gemisch oder nacheinander in Gegenwart von Säuren oder vorzugsweise von starken Basen als Katalysatoren an Starterkomponenten mit reaktiven Wasserstoffatomen hergestellt. Beispiele für derartige Starterkomponenten sind n-Butanol, n-Hexanol, Phenol, Wasser, Ethylenglykol, 1,2- und 1,3-Propylenglykol, 1,4-Butandiol, 4,4'-Dihydroxy-diphenylpropan, Glycerin, Trimethylolpropan, Erythrit, Sorbit, Ammoniak, Ethylendiamin, Anilin, Ethanolamin und Triethanolamin. Auch Sucrosepolyether, wie sie z.B. in der deutschen Auslegeschrift 1 176 358 und 1 064 938 beschrieben werden, und durch Vinylpolymerisate modifizierte Polyether, wie sie z.B. durch Polymerisation von Styrol und Acrylnitril in Gegenwart von Polyethern entstehen (US 3 383 351, 3 304 273, 3 523 093, 3 110 695, DE-B 1 152 536) können erfindungsgemäß gereinigt werden. Bevorzugt sind Polyetherpolyole auf Basis von Ethylenoxid und/oder Propylenoxid mit 1 bis 8, bevorzugt 2 bis 6 Hydroxylgruppen, deren Molekulargewichte und Viskositäten in dem oben angegebenen Bereich liegt.

Ein weiterer Gegenstand der vorliegenden Erfindung ist ein Verfahren zur Herstellung der geruchsarmen, höhermolekularen Polyetherpolyole, das dadurch gekennzeichnet ist, daß man die zu reinigenden Polyetherpolyole unter Zugabe von 5,0 bis 30,0 Gew.-%, bevorzugt 7,0 bis 25,0 Gew.-%, Wasser, bezogen auf die eingesetzten Polyetherpolyole, bei Temperaturen von 110 bis 150°C, bevorzugt 115 bis 140°C, und einem Druck von 10 bis 70 hPa, bevorzugt 20 bis 50 hPa destilliert, wobei man das Wasser in einer Dosierzeit von 1 h bis 5 h, bevorzugt 2 h bis 4 h, in feinverteilter Form mit einem Tropfendurchmesser von 5,0 bis 100 µ, bevorzugt 7 bis 50 µ, durch die zu reinigenden Polyetherpolyole leitet.

Für das erfindungsgemäße Verfahren ist es wichtig, daß während der Destillation der zu reinigenden Polyetherpolyole die Zudosierung des Wassers so erfolgt, daß die untere Temperaturgrenze bei der Destillation nicht unterschritten und die obere Druckgrenze nicht überschritten wird. Dies wird insbesondere durch die vorgegebene Dosierzeit des in feinverteilter Form den zu reinigenden Polyetherpolyolen zudosierten Wassers bewerkstelligt. Werden die vorgegebenen Destillationsbedingungen nicht eingehalten, so werden die geruchsbildenden Stoffe in den Polyetherpolyolen nur unzureichend entfernt.

Wie erwähnt, ist es für das erfindungsgemäße Verfahren ebenfalls von Bedeutung, daß das Wasser in Form von sehr kleinen Tropfen in die Polyetherpolyole eingebracht wird. Dies kann beispielsweise mittels eines Kapillarrohrs, einer feinen Keramikfritte, einer gesinterten Metallfritte oder mittels feiner Druckdüsen oder pneumatischer Zweistoffdüsen erfolgen.

Damit das eingeleitete Wasser mit den zu reinigenden Polyetherpolyolen intensiv in Kontakt kommt, ist eine gute Durchmischung des Destillationsgemisches angezeigt. Hierzu können die bekannten Mischeinrichtungen, wie Kreuzbalkenrührer, Gitterrührer oder Ultrathoraxmischer, verwendet werden.

Das erfindungsgemäße Verfahren kann den bekannten Polyetherpolyol-Herstellungsverfahren als separater Reinigungsschritt nachgeschaltet werden. Es ist selbstverständlich auch möglich, die im Handel erhältlichen Polyetherpolyole einer Nachreinigung mit dem erfindungsgemäßen Verfahren zu unterwerfen, um die handelsüblichen Polyetherpolyole in praktisch geruchsfreier Form zu erhalten.

Die erfindungsgemäßen, geruchsarmen Polyetherpolyole können zur Herstellung von auf Polyetherpolyolen aufgebauten emissionsarmen Polymeren, wie Polyurethanen, insbesondere Polyurethan-Weichschäumen, Elastomeren oder für die Herstellung von Kosmetika und pharmazeutischen Produkten verwendet werden.

**Beispiele**

In den nachfolgenden Beispielen wurden 1 000 g der verschiedensten Polyetherpolyole auf 120°C aufgeheizt. Bei einem Druck von 18 hPa wurden 200 g Wasser (20 Gew.-%) über dünne Einleitungsrohre (Durchmesser: 2 mm) mit einer solchen Geschwindigkeit eingeleitet, daß die Temperatur von 120°C nicht unterschritten wurde und der Druck nicht über 40 hPa anstieg. Die Dosierzeit des zudosierten Wassers betrug 3 h. Die Wasserblasen hatten einen mittleren Durchmesser von 7 bis 50 µ. In den Beispielen wurden Di- und trifunktionelle PO- und PO/EO-Polyether eingesetzt. Aus Tabelle 1 ist ersichtlich wie sich die erfindungsgemäße Destillation auf die Verunreinigungen in den eingesetzten Polyetherpolyolen auswirkt. Es ist klar zu erkennen, daß nach der erfindungsgemäßen Destillation die unerwünschten Nebenprodukte, die u.a. zur Geruchsbildung führen, weitestgehend entfernt wurden. Die Analyse wurde mittels Headspace-Gaschromatographie durchgeführt.

Tabelle 1

| Verbindungen in den Polyether-polyolen | Beispiel 1 | | Beispiel 2 | |
|---|---|---|---|---|
| | vor Destillation | nach Destillation | vor Destillation | nach Destillation |
| 1,4-Dioxan | < 0,1, | < 0,1 | < 0,1 | < 0,1 |
| 2,4-Dimethyl-1,3-Dioxolan | 1,5 | < 0,1 | < 0,1 | < 0,1 |
| 2-Ethylen-4-methyl-1,3-dioxolon | 2,5 | < 0,1 | < 0,1 | < 0,1 |
| 2-Methyl-2-pentenal | 1,8 | < 0,1 | 2,2 | < 0,1 |
| Acetaldehyd | 1,2 | 0,1 | 1,9 | 2 |
| Allylalkohol | < 0,1 | < 0,1 | 0,6 | < 0,1 |
| Allyloxipropanol | 80 | < 0,1 | 0,6 | < 0,1 |
| Butyraldehyd | < 0,1 | < 0,1 | < 0,1 | < 0,1 |
| DPG-Allylether | 460 | < 0,1 | 480 | < 0,1 |
| Propionaldehyd | 1,1 | 0,1 | 0,4 | 0,3 |
| Summe unbek. leicht flüchtiger Verbindungen | 20 | 8 | 15 | 8 |
| Verbindungen in den Polyether-polyolen | Beispiel 3 | | Beispiel 4 | |
| | vor Destillation | nach Destillation | vor Destillation | nach Destillation |
| 1,4-Dioxan | < 0,1, | < 0,1 | < 0,1 | < 0,1 |
| 2,4-Dimethyl-1,3-Dioxolan | < 0,1 | < 0,1 | < 0,1 | < 0,1 |
| 2-Ethylen-4-methyl-1,3-dioxolon | 1,2 | < 0,1 | < 0,1 | < 0,1 |
| 2-Methyl-2-pentenal | 6 | 0,3 | 0,3 | < 0,1 |
| Acetaldehyd | 0,5 | 0,8 | 0,6 | 1,3 |
| Allylalkohol | 1,8 | < 0,1 | < 0,1 | < 0,1 |
| Allyloxipropanol | 170 | 1,4 | 12 | 0,3 |
| Butyraldehyd | < 0,1 | < 0,1 | < 0,1 | < 0,1 |
| DPG-Allylether | 650 | < 0,1 | 55 | < 0,1 |
| Propionaldehyd | 2,7 | 0,1 | 0,3 | 0,3 |
| Summe unbek. leicht flüchtiger Verbindungen | 8 | 8 | 8 | 4 |
| (alle Angaben in ppm) | | | | |

Der Polyether in Beispiel 1 war ein verzweigter Polyether auf Basis Glycerin, Ethylenoxid und Propylenoxid, OHZ 46 (= OH-Zahl) Molekulargewicht (Mw): 3660, Viskosität: 560 mPa·s

Der Polyether in Beispiel 2 war ein linearer Polyether auf Basis Propylenglykol und PO, OHZ 112, Mw: 1000, Viskosität: 140 mP·s.

Der Polyether in Beispiel 3 war ein linearer Polyether auf Basis Propylenglykol und PO, OHZ 56, Mw: 2000, Viskosität: 310 mP·s.

Der Polyether in Beispiel war ein verzweigter Polyether auf Basis Trimethylolpropan, PO und EO, OHZ 28, Mw: 6000, Viskosität: 1120 mPa·s.

EO =    Ethylenoxid

PO =     Propylenoxid

**Patentansprüche**

1.  Geruchsarme, monofunktionelle und polyfunktionelle Polyetherpolyole, deren Molekulargewicht bei 750 bis 18 000, bevorzugt bei 1 000 bis 15 000, ganz besonders bevorzugt bei 2 000 bis 12 000, liegt (berechnet nach OH-Zahl und Funktionalität:

$$\text{Mol Gew.} = \frac{F\ OH.56.100}{OH\text{-Zahl}})$$

und deren Viskosität bei 25°C, bestimmt mit Höpplerviskosimeter nach DIN 53015, 40 bis 25 000, vorzugsweise 50 bis 10 000 mPa·s beträgt, die dadurch gekennzeichnet sind, daß deren Gehalt an

a) 2-Methyl-2-Pentenal < 1,5 ppm, bevorzugt < 0,2 ppm,

b) Allylalkohol < 1,0 ppm, bevorzugt < 0,2 ppm,

c) Allyloxypropanol < 15 ppm, bevorzugt < 5,0 ppm,

d) Dipropylenglykolallylether < 50 ppm, bevorzugt < 0,5 ppm,

e) Propionaldehyd < 1,0 ppm, bevorzugt < 0,2 ppm

beträgt.

2.  Verfahren zur Herstellung von Polyetherpolyolen nach Anspruch 1, dadurch gekennzeichnet, daß man die zu reinigenden Polyetherpolyole unter Zugabe von 5 bis 30 Gew.-% Wasser, bezogen auf die eingesetzten Polyetherpolyole, bei Temperaturen von 110 bis 150°C und Drücken von 10 bis 70 hPa destilliert, wobei man das Wasser in einer Dosierzeit von 1 bis 5 Stunden in feinverteilter Form mit einem Tropfendurchmesser von 5 bis 100 μ durch die zu reinigenden Polyetherpolyole leitet.

3.  Verwendung der geruchsarmen Polyetherpolyole nach Anspruch 1 zur Herstellung von auf Polyetherpolyolen aufbauenden emissionsarmen Polymeren, Kosmetika und pharmazeutischen Produkten.